(19) Europäisches Patentamt
European Patent Office
Office européen des brevets

(11) Numéro de publication : 0 436 410 A1

(12) DEMANDE DE BREVET EUROPEEN

(21) Numéro de dépôt : 90403317.2

(22) Date de dépôt : 23.11.90

(51) Int. Cl.[5] : C07C 37/01, C07C 39/08, C07C 45/46, C07C 49/825, C07C 39/15, C07C 39/17

(30) Priorité : 05.12.89 FR 8916310

(43) Date de publication de la demande : 10.07.91 Bulletin 91/28

(84) Etats contractants désignés : AT BE CH DE DK ES FR GB GR IT LI LU NL SE

(71) Demandeur : RHONE-POULENC CHIMIE
25, quai Paul Doumer
F-92408 Courbevoie Cédex (FR)

(72) Inventeur : Bonneau, Isabelle, Résidence Charrière Blanche
Chemin de Charrière Blanche
F-69130 Ecully (FR)
Inventeur : Rochin, Christophe
21, rue Dussaussoy
F-69006 Lyon (FR)

(74) Mandataire : Dutruc-Rosset, Marie-Claude et al
RHONE-POULENC INTERSERVICES Service Brevets Chimie 25, Quai Paul Doumer
F-92408 Courbevoie Cédex (FR)

# (54) Procédé de préparation d'hydroxyquinones substituées.

(57) La présente invention concerne un procédé de préparation d'hydroquinones monosubstituées caractérisé en ce que l'on fait réagir un phénol substitué en position ortho ou méta par rapport au groupement OH, avec un agent d'acétylation choisi parmi les halogènures d'acétyle, l'acide acétique, l'anhydride acétique ou les esters de l'acide acétique, dans le fluorure d'hydrogène, afin de former l'hydroxyacétophénone R-substituée correspondante et l'on oxyde ladite hydroxyacétophénone par le peroxyde d'hydrogène en présence d'une base minérale.

Le procédé de l'invention est plus particulièrement intéressant pour préparer la méthylhydroquinone, en raison du faible coût de l'ortho-crésol ou du méta-crésol servant de matière première.

EP 0 436 410 A1

# PROCEDE DE PREPARATION D'HYDROQUINONES SUBSTITUEES

La présente invention concerne un procédé de préparation d'hydroquinones monosubstituées.

Les hydroquinones monosubstituées, notamment par des radicaux hydrocarbonés peuvent servir notamment pour la préparation de monomères. C'est en particulier le cas de la méthylhydroquinone qui est le composé le plus connu de cette famille.

De très nombreuses voies d'accès à la méthylhydroquinone ont été proposées.

Le brevet américain n°2 041 593 décrit l'hydrolyse du chloro-4 méthyl-2 phénol en méthylhydroquinone, en milieu aqueux sodique. Ce procédé présente une mauvaise sélectivité en méthylhydroquinone.

Le brevet EP-A-0 041 441 décrit l'hydroxylation de l'orthocrésol dans le fluorure d'hydrogène à -40°C, en présence de pentafluorure d'antimoine. Malgré des conditions opératoires très dures, es rendements sont insuffisants.

Le brevet américain n°4 482 756 préconise l'oxydation de l'orthocrésol par l'oxygène, en présence de chlorure cuivrique dans l'acétonitrile. Ce procédé nécessite la mise en oeuvre de pressions élevées.

La présente invention concerne un procédé de préparation d'hydroquinones monosubstituées de formule générale (I) :

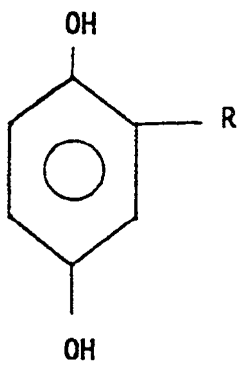

(I)

dans laquelle R représente :

- un radical alkyle, linéaire ou ramifié, ayant 1 à 4 atomes de carbone ;
- un radical phényle ;
- un radical cyclohexyle ;

caractérisé en ce que :

- l'on fait réagir un phénol substitué de formule générale (II) :

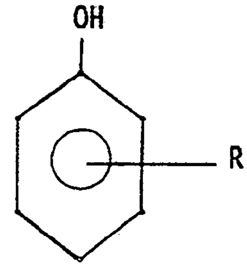

(II)

dans laquelle R a les significations indiquées pour la formule (I) et se trouve en position ortho ou méta par rapport au groupement OH,

avec un agent d'acétylation choisi parmi les halogènures d'acétyle, l'acide acétique, l'anhydride acétique ou les esters de l'acide acétique, dans le fluorure d'hydrogène,

afin de former l'hydroxyacétophénone R-substituée correspondante,

- et l'on oxyde ladite hydroxyacétophénone par le peroxyde d'hydrogène en présence d'une base minérale.

Parmi les composés de formule (I) qui sont actuellement considérés comme les plus intéressants on peut citer la méthylhydroquinone, l'éthylhydroquinone, la cyclohexylhydroquinone et la phénylhydroquinone.

Le procédé de l'invention est plus particulièrement intéressant pour préparer la méthylhydroquinone, en raison du faible coût de l'ortho-crésol ou du méta-crésol servant de matière première.

L'agent d'acétylation le plus pratique à utiliser dans la présente invention est un halogénure d'acétyle, car le sous-produit de la réaction est alors un halogénure d'hydrogène, qui ne perturbe pas le traitement final, ni le recyclage du fluorure d'hydrogène. Parmi ces halogénures, on préfère utiliser le chlorure d'acétyle, le chlorure d'hydrogène qui se forme se séparant facilement du milieu réactionnel.

La réaction d'acétylation selon l'invention est réalisée dans le fluorure d'hydrogène, de préférence anhydre.

La quantité d'agent d'acétylation est telle que le rapport molaire agent d'acétylation/phénol R-substitué de formule (II) est habituellement de 1 à 5.

De préférence ce rapport molaire sera de 1 à 2.

La quantité de fluorure d'hydrogène utilisée est telle que le rapport molaire HF/phénol de formule (II) est généralement compris entre 5 et 50.

La température à laquelle est conduite la réaction d'acétylation varie largement. Généralement elle se situe entre -10°C et 120°C.

De préférence cette température est comprise entre 20°C et 100°C.

La pression dans le réacteur est généralement la

pression autogène des différents réactifs ou solvants à la température choisie.

La réaction d'acétylation peut être réalisée de manière pratique en chargeant dans un réacteur le fluorure d'hydrogène anhydre à l'état liquide, généralement à une température de 0°C à 10°C, puis le phénol substitué de formule (II).

L'agent d'acétylation, de préférence le chlorure d'acétyle, est alors introduit progressivement.

Après la fin d'addition de l'agent d'acétylation, le réacteur est fermé et le mélange réactionnel est chauffé à la température choisie, pendant une durée de quelques minutes à plusieurs heures, sous pression autogène.

En fin de réaction d'acétylation, l'hydroxyacétophénone formée peut être séparée par les méthodes usuelles de la chimie, par exemple par extractions, décantations, évaporations.

On peut également dans la présente invention simplement éliminer, par exemple par distillation, le fluorure d'hydrogène et engager l'hydroxyacétophénone brute dans l'étape d'oxydation.

La réaction d'oxydation de l'hydroxyacétophénone substituée en hydroquinone de formule (I) s'effectue avec un rapport molaire peroxyde d'hydrogène/hydroxyacétophénone qui se situe généralement entre 1 et 2.

Pour des questions économiques, il n'est pas utile de mettre un important excès de peroxyde d'hydrogène et le plus souvent ce rapport se situe entre 1 et 1,2.

Le peroxyde d'hydrogène est habituellement utilisé sous la forme des solutions aqueuses disponibles dans le commerce, qui ont une concentration allant le plus souvent de 20% à 70%.

La base minérale utilisée dans la réaction d'oxydation est de préférence un hydroxyde de métal alcalin et plus particulièrement la soude.

Le rapport global molaire base minérale/hydroxyacétophénone substituée se situe généralement entre 1 et 1,2.

La base minérale est généralement mise en oeuvre sous forme d'une solution aqueuse.

Le pH du milieu réactionnel est maintenu pendant la réaction d'oxydation à une valeur située entre 8 et 11 et de préférence entre 8,5 et 10,5.

Le contrôle s'effectue par mesure du pH et addition progressive de la base minérale pendant la réaction.

Le solvant utilisé dans l'opération est l'eau. On peut également utiliser éventuellement un tiers solvant inerte dans les conditions réactionnelles.

A titre d'exemples non limitatifs de tels tiers solvants, on peut citer les alcools tels que notamment le méthanol.

La concentration dans le mélange réactionnel, en hydroxyacétophénone substituée engagée et/ou en hydroquinone substituée formée dans la réaction, n'est pas critique. Habituellement la concentration en hydroxyacétophénone substituée engagée se situe entre 10% et 60% en poids par rapport au poids total du mélange réactionnel. De préférence cette concentration pondérale est de 30% à 50%.

La température à laquelle est réalisée la réaction d'oxydation est le plus souvent de 10°C à 60°C. De préférence, elle se situe entre 25°C et 35°C.

Un mode pratique de réalisation de la réaction d'oxydation consiste à charger l'hydroxyacétophénone substituée obtenue dans la réaction d'acétylation avec de l'eau et éventuellement une partie de la base minérale pour ajuster le pH du mélange à la valeur désirée.

Le mélange est agité et chauffé entre 10°C et 60°C et une solution de peroxyde d'hydrogène et une solution de base minérale sont coulées simultanément, mais indépendamment l'une de l'autre. Le pH du mélange réactionnel et la température sont maintenues aux valeurs choisies.

Après la fin d'addition du peroxyde d'hydrogène et de la base minérale, le mélange réactionnel est maintenu sous agitation à la température choisie, pendant une durée variable de quelques minutes à quelques heures.

Le mélange réactionnel final est traité par les méthodes usuelles de la chimie, afin d'isoler l'hydroquinone substituée ainsi préparée.

Le procédé de l'invention peut être mis en oeuvre de manière discontinue ou de manière continue.

Les exemples qui suivent illustrent l'invention.

## EXEMPLE 1

### A) Acétylation de l'ortho-crésol en hydroxy-4 méthyl-3 acétophénone

Dans un réacteur en Hastelloy de 250 $cm^3$, on charge à 0°C :

- fluorure d'hydrogène (HF) : 100 g (5 mol)
- ortho-crésol : 21,6 g (0,2 mol)

On introduit 15,7 g (0,2 mol) de chlorure d'acétyle en 1 h 20 min, en dégageant le chlorure d'hydrogène qui se forme. On ferme ensuite le réacteur et on chauffe 1 heure à 50°C (la pression autogène est de 0,3 MPa).

On refroidit le réacteur, on évapore environ 85% du fluorure d'hydrogène sous pression réduite.

On verse le mélange réactionnel ainsi obtenu dans un récipient contenant 300 g de glace et on rince le réacteur par 3 fois 100 $cm^3$ de dichlorométhane.

Après décantation et séparation, on lave la phase organique par 2 fois 100 $cm^3$ d'eau, puis on la sèche sur sulfate de sodium.

Après évaporation du solvant, on obtient 29,8 g d'un solide beige clair, titrant par chromatographie liquide haute performance (CLHP) 98% en hydroxy-4 méthyl-3 acétophénone.

Après recristallisation, on obtient 26 g d'hydroxy-4 méthyl-3 acétophénone pure, dont la structure est confirmée par résonance magnétique nucléaire (RMN).

On obtient les résultats suivants :

- taux de transformation (TT) de l'ortho-crésol : 99%
- rendement en hydroxy-4 méthyl-3 acétophénone dosée, par rapport à l'ortho-crésol transformé : 98%
- rendement en hydroxy-2 méthyl-3 acétophénone dosée, par rapport à l'ortho-crésol transformé : moins de 2%

B) Oxydation de l'hydroxy-4 méthyl-3 acétophénone en méthylhydroquinone

Dans un réacteur en verre de 50 $cm^3$, muni d'une agitation centrale, on charge sous courant d'argon :

- hydroxy-4 méthyl-3 acétophénone pure préparée en A) : 6 g (0,04 mol)
- eau : 8 $cm^3$
- soude à 35% en poids par poids (p/p) : 0,7 $cm^3$ (0,008 mol)

On agite et on chauffe à 30°C. On maintient cette température pendant toute la durée de la réaction.

On coule simultanément en 1 h, par 2 arrivées distinctes, 3,5 $cm^3$ (0,040 mol) d'une solution aqueuse de peroxyde d'hydrogène à 35% p/p et 3,3 $cm^3$ (0,032 mol) de soude à 35% p/p. Le pH du mélange réactionnel varie durant ces coulées de 10,5 à 9.

L'agitation et la température sont maintenues ensuite pendant 2 h 30.

On ajoute ensuite 2,2 $cm^3$ d'une solution 1 M de sulfite de sodium au mélange réactionnel final.

On extrait à l'aide d'acétate d'éthyle le mélange réactionnel.

La phase organique est analysée par chromatographie liquide haute performance (CLHP) et par potentiométrie, la structure chimique des composés étant confirmée par spectométrie de masse.

On obtient les résultats suivants :

- TT% de l'hydroxy-4 méthyl-3 acétophénone (HMA) : 95%
- Rendement (RT) en méthylhydroquinone (MeHQ) par rapport à l'HMA transformée : 90%
- RT% en acide hydroxy-4 méthyl-3 benzoïque : 9%

EXEMPLE 2

A) Acétylation de l'ortho-crésol en hydroxy-4 méthyl-3 acétophénone

Dans un réacteur en Hastelloy de 250 $cm^3$, on charge à 0°C :

- fluorure d'hydrogène (HF) : 100 g (5 mol)
- ortho-crésol : 21,6 g (0,2 mol)

On introduit 15,7 g (0,2 mol) de chlorure d'acétyle en 1 h, en dégageant le chlorure d'hydrogène qui se forme. On ferme ensuite le réacteur et on chauffe 2 heure à 40°C.

On refroidit le réacteur, on évapore environ 85% du fluorure d'hydrogène sous pression réduite.

On verse le mélange réactionnel ainsi obtenu dans un récipient contenant 300 g de glace et on rince le réacteur par 100 $cm^3$ de chloroforme.

Après décantation et séparation, on lave la phase organique par 2 fois 100 $cm^3$ d'eau, puis on la sèche sur sulfate de sodium.

Après évaporation du solvant, on obtient 29,4 g d'un solide beige clair, titrant par chromatographie liquide haute performance (CLHP) 98% en hydroxy-4 méthyl-3 acétophénone.

Après recristallisation, on obtient 27,3 g d'hydroxy-4 méthyl-3 acétophénone pure, dont la structure est confirmée par résonance magnétique nucléaire (RMN).

On obtient les résultats suivants :

- taux de transformation (TT) de l'ortho-crésol : 99%
- rendement en hydroxy-4 méthyl-3 acétophénone dosée, par rapport à l'ortho-crésol transformé : 97%
- rendement en hydroxy-2 méthyl-3 acétophénone dosée, par rapport à l'ortho-crésol transformé : moins de 2%

B) Oxydation de l'hydroxy-4 méthyl-3 acétophénone en méthylhydroquinone

Dans un réacteur en verre de 50 $cm^3$, muni d'une agitation centrale, on charge sous courant d'argon :

- hydroxy-4 méthyl-3 acétophénone pure préparée en A) : 6 g (0,04 mol)
- eau : 4 $cm^3$
- méthanol : 4 $cm^3$

On agite et on chauffe à 30°C. On maintient cette température pendant toute la durée de la réaction.

On coule simultanément par 2 arrivées distinctes, 3,5 $cm^3$ (0,040 mol) d'une solution aqueuse de peroxyde d'hydrogène à 35% p/p et 3,4 $cm^3$ (0,040 mol) de soude à 35% p/p.

La durée de coulée du peroxyde d'hydrogène est d'une heure.

La coulée de la solution de soude est asservie au pH du mélange réactionnel régulé automatiquement à 9.

L'agitation et la température sont maintenues au total pendant 3 h 30.

On ajoute ensuite 2,2 $cm^3$ d'une solution 1 M de sulfite de sodium au mélange réactionnel final.

On extrait à l'aide d'acétate d'éthyle le mélange réactionnel.

La phase organique est analysée par chromatographie liquide haute performance (CLHP) et par potentiométrie, la structure chimique des composés étant confirmée par spectométrie de masse.

On obtient les résultats suivants :

- TT% de l'hydroxy-4 méthyl-3 acétophénone (HMA) : 96%
- Rendement (RT) en méthylhydroquinone (MeHQ) par rapport à l'HMA transformée : 99%

EXEMPLE 3 : oxydation de l'hydroxy-4 méthyl-3 acétophénone en méthylhydroquinone

Dans un réacteur en verre de 50 $cm^3$, muni d'une agitation centrale, on charge sous courant d'argon :

- hydroxy-4 méthyl-3 acétophénone pure préparée dans l'exemple 2 A) : 6 g (0,04 mol)
- eau : 4 $cm^3$
- méthanol : 4 $cm^3$

On agite et on chauffe à 30°C. On maintient cette température pendant toute la durée de la réaction.

On coule simultanément par 2 arrivées distinctes, 3,5 $cm^3$ (0,040 mol) d'une solution aqueuse de peroxyde d'hydrogène à 35% p/p et 3,4 $cm^3$ (0,040 mol) de soude à 35% p/p.

La durée de coulée du peroxyde d'hydrogène est d'une heure.

La coulée de la solution de soude est asservie au pH du mélange réactionnel régulé automatiquement à 10.

L'agitation et la température sont maintenues au total pendant 3 h 30.

On ajoute ensuite 2,2 $cm^3$ d'une solution 1 M de sulfite de sodium au mélange réactionnel final.

On extrait à l'aide d'acétate d'éthyle le mélange réactionnel.

La phase organique est analysée par chromatographie liquide haute performance (CLHP) et par potentiométrie, la structure chimique des composés étant confirmée par spectométrie de masse.

On obtient les résultats suivants :

- TT% de l'hydroxy-4 méthyl-3 acétophénone HMA : 83%
- Rendement (RT) en méthylhydroquinone (MeHQ) par rapport à l'HMA transformée : 83%

EXEMPLE 4 : oxydation de l'hydroxy-4 méthyl-3 acétophénone en méthylhydroquinone

Dans un réacteur en verre de 50 $cm^3$, muni d'une agitation centrale, on charge sous courant d'argon :

- hydroxy-4 méthyl-3 acétophénone pure préparée dans l'exemple 2 A) : 6 g (0,04 mol)
- eau : 8 $cm^3$
- soude à 35% en poids par poids (p/p) : 0,7 $cm^3$ (0,008 mol)

On agite et on chauffe à 30°C. On maintient cette température pendant toute la durée de la réaction.

On coule simultanément en 1 h, par 2 arrivées distinctes, 3,5 $cm^3$ (0,040 mol) d'une solution aqueuse de peroxyde d'hydrogène à 35% p/p et 3,3 $cm^3$ (0,032 mol) de soude à 35% p/p. Le pH du mélange réactionnel varie durant ces coulées de 10,5 à 9.

La température est montée à 40°C et est maintenue sous agitation pendant encore 2 h 30.

On ajoute ensuite 2,2 $cm^3$ d'une solution 1 M de sulfite de sodium au mélange réactionnel final.

On extrait à l'aide d'acétate d'éthyle le mélange réactionnel.

La phase organique est analysée par chromatographie liquide haute performance (CLHP) et par potentiométrie, la structure chimique des composés étant confirmée par spectométrie de masse.

On obtient les résultats suivants :

- TT% de l'hydroxy-4 méthyl-3 acétophénone (HMA) : 90%
- Rendement (RT) en méthylhydroquinone (MeHQ) par rapport à l'HMA transformée : 88%

EXEMPLE 5 : oxydation de l'hydroxy-4 méthyl-3 acétophénone en méthylhydroquinone

Dans un réacteur en verre de 50 $cm^3$, muni d'une agitation centrale, on charge sous courant d'argon :

- hydroxy-4 méthyl-3 acétophénone brute préparée dans l'exemple 1 A) : 6,12 g (0,04 mol)
- eau : 4 $cm^3$
- méthanol : 4 $cm^3$

On agite et on chauffe à 30°C. On maintient cette température pendant toute la durée de la réaction.

On coule simultanément par 2 arrivées distinctes, 3,5 $cm^3$ (0,040 mol) d'une solution aqueuse de peroxyde d'hydrogène à 35% p/p et 3,4 $cm^3$ (0,040 mol) de soude à 35% p/p.

La durée de coulée du peroxyde d'hydrogène est d'une heure.

La coulée de la solution de soude est asservie au pH du mélange réactionnel régulé automatiquement à 9.

L'agitation et la température sont maintenues au total pendant 3 h 30.

On ajoute ensuite 2,2 $cm^3$ d'une solution 1 M de sulfite de sodium au mélange réactionnel final.

On extrait à l'aide d'acétate d'éthyte le métange réactionnel.

La phase organique est analysée par chromatographie liquide haute performance (CLHP) et par potentiométrie, la structure chimique des composés étant confirmée par spectométrie de masse.

On obtient les résultats suivants :

- TT% de l'hydroxy-4 méthyl-3 acétophénone (HMA) : 95%
- Rendement (RT) en méthylhydroquinone (MeHQ) par rapport à l'HMA transformée : 86%

## Revendications

1. Procédé de préparation d'hydroquinones monosubstituées de formule générale (I) :

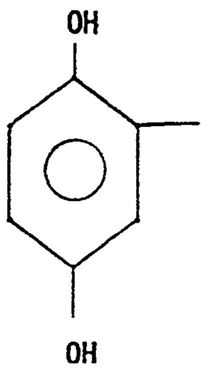

(I)

dans laquelle R représente :
• un radical alkyte, linéaire ou ramifié, ayant 1 à 4 atomes de carbone ;
• un radical phényle ;
• un radical cyclohexyle ;
caractérisé en ce que :
- l'on fait réagir un phénol substitué de formule générale (II) :

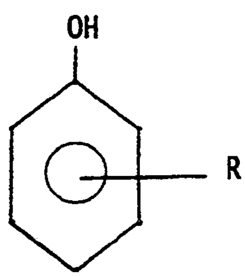

(II)

dans laquelle R a les significations indiquées pour la formule (I) et se trouve en position ortho ou méta par rapport au groupement OH, avec un agent d'acétylation choisi parmi les halogènures d'acétyle, l'acide acétique, l'anhydride acétique ou les esters de l'acide acétique, dans le fluorure d'hydrogène,
afin de former l'hydroxyacétophénone R-substituée correspondante,
- et l'on oxyde ladite hydroxyacétophénone par le peroxyde d'hydrogène en présence d'une base minérale.

2. Procédé selon la revendication 1, caractérisé en ce que le composé de formule (I) est choisi parmi la méthylhydroquinone, l'éthylhydroquinone, la cyclohexylhydroquinone et la phénylhydroquinone.

3. Procédé selon l'une des revendications 1 ou 2, caractérisé en ce que l'agent d'acétylation est un halogénure d'acétyle et de préférence le chlorure d'acétyle.

4. Procédé selon l'une des revendications 1 à 3, caractérisé en ce que la quantité d'agent d'acétylation est telle que le rapport molaire agent d'acétylation/phénol R-substitué de formule (II) est de 1 à 5 et de préference de 1 à 2.

5. Procédé selon l'une des revendications 1 à 4, caractérisé en ce que la quantité de fluorure d'hydrogène utilisée est telle que le rapport molaire HF/phénol de formule (II) est compris entre 5 et 50.

6. Procédé selon l'une des revendications 1 à 5, caractérisé en ce que la température à laquelle est conduite la réaction d'acétylation se situe entre -10°C et 120°C, et de préference entre 20°C et 100°C.

7. Procédé selon l'une des revendications 1 à 6, caractérisé en ce que la réaction d'oxydation de l'hydroxyacétophénone substituée en hydroquinone de formule (I) s'effectue avec un rapport molaire peroxyde d'hydrogène/hydroxyacétophénone qui se situe entre 1 et 2 et de préférence entre 1 et 1,2.

8. Procédé selon l'une des revendications 1 à 7, caractérisé en ce que la base minérale utitisée dans la réaction d'oxydation est un hydroxyde de métal alcalin et de préférence la soude.

9. Procédé selon l'une des revendications 1 à 8, caractérisé en ce que le rapport global molaire base minérale/hydroxyacétophénone substituée se situe entre 1 et 1,2.

10. Procédé selon l'une des revendications 1 à 9, caractérisé en ce que le pH du milieu réactionnel est maintenu pendant la réaction d'oxydation à une valeur située entre 8 et 11 et de préférence entre 8,5 et 10,5.

11. Procédé selon l'une des revendications 1 à 10, caractérisé en ce que la concentration en hydroxyacétophénone substituée engagée se situe entre 10% et 60% en poids par rapport au poids total du mélange réactionnel, et de préférence entre 30% à 50%.

12. Procédé selon l'une des revendications 1 à 11, caractérisé en ce que la température à laquelle est réalisée la réaction d'oxydation est de 10°C à 60°C et de préférence de 25°C et 35°C.

13. Procédé selon l'une des revendications 1 à 12, caractérisé en ce qu'après la réaction d'acétylation on élimine le fluorure d'hydrogène et on engage l'hydroxyacétophénone brute dans l'étape d'oxydation.

Office européen des brevets

# RAPPORT DE RECHERCHE EUROPEENNE

Numero de la demande

EP 90 40 3317
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y | EP-A-178929 (CELANESE CORP.)<br>* p. 6, l. 13-24; p. 7, l. 14 - p. 8, l. 22; rev. 1 * | 1-6, 8, 12, 13 | C07C37/01<br>C07C39/08<br>C07C45/46<br>C07C49/825<br>C07C39/15<br>C07C39/17 |
| Y | Houben -Weyl: "Methoden der organischen Chemie. 4. éd. tome VI/1c, part. 1,2"<br>1976, Georg Thieme Verlag, Stuttgart, DE<br>* part. 1, p. 286, l. 18-22; p. 292, para. 3; part. 2, p. 1093 * | 1-6, 8, 12, 13 | |
| A | Theilheimer's Synthetic Methods of Organic Chemistry<br>vol. 30, 1976, Basel, CH<br>page 79 & H. Bretschneider: "Helvetica Chemica Acta, vol. 56, 1973, p. 2857"<br>* page 79, alinéa 1 * | 1 | |
| A | US-A-3764629 (H. GURIEN ET AL.)<br>* exemple 1; revendication 1 * | 1 | |
| A | EP-A-320346 (RHONE-POULENC)<br>* page 6, exemple 36 * | 1 | **DOMAINES TECHNIQUES RECHERCHES (Int. Cl.5 )** |
| A | PATENT ABSTRACTS OF JAPAN<br>vol. 6, no. 24 (C-91)(902) 12 février 1982,<br>& JP-A-56 147737 (SUMITOMO) 16 novembre 1981,<br>* le document en entier * | 1 | C07C |
| A | PATENT ABSTRACTS OF JAPAN<br>vol. 11, no. 349 (C-456)(2796) 14 novembre 1987,<br>& JP-A-62 120334 (DAINIPPON INK & CHEMICAL) 01 juin 1987,<br>* le document en entier * | 1 | |

Le présent rapport a été établi pour toutes les revendications

| Lieu de la recherche | Date d'achèvement de la recherche | Examinateur |
|---|---|---|
| BERLIN | 11 MARS 1991 | PROBERT,C |

2

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 90 40 3317
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | CHEMICAL ABSTRACTS, vol. 108, no. 5, 01 février 1988<br>Columbus, Ohio, USA<br>S. Nakatsuka et al.: "Introduction of a hydroxy group into the 5- and 6-positions of the indole nucleus by Friedel-Crafts acylation ..."<br>page 610; colonne de gauche; ref. no. 37574Q & HETEROCYCLES 1987 26[6] 1471-4<br>* abrégé *<br>----- | 1 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| BERLIN | 11 MARS 1991 | PROBERT,C |

2

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)